(19) Europäisches Patentamt
European Patent Office
Office européen des brevets

(11) EP 1 375 537 B1

(12) # EUROPÄISCHE PATENTSCHRIFT

(45) Veröffentlichungstag und Bekanntmachung des Hinweises auf die Patenterteilung:
**21.04.2010 Patentblatt 2010/16**

(51) Int Cl.:
***C08F 20/04*** *(2006.01)* ***C08F 251/00*** *(2006.01)*
***A61L 15/00*** *(2006.01)*

(21) Anmeldenummer: **03021645.1**

(22) Anmeldetag: **28.05.1999**

(54) **Wasserabsorbierende Polymere mit supermolekularen Hohlraummolekülen, Verfahren zu deren Herstellung und deren Verwendung**

Water-absorbing polymers with supramolecular hollow molecules, method for producing them and use of the same

Polymères hydroabsorbants avec des molecules creuses supramoléculaires, leur procédé de préparation et leur utilisation

(84) Benannte Vertragsstaaten:
**AT BE CH CY DE DK ES FI FR GB GR IE IT LI LU MC NL PT SE**

(30) Priorität: **08.06.1998 DE 19825486**

(43) Veröffentlichungstag der Anmeldung:
**02.01.2004 Patentblatt 2004/01**

(62) Dokumentnummer(n) der früheren Anmeldung(en) nach Art. 76 EPÜ:
**99926461.7 / 1 091 983**

(73) Patentinhaber: **Evonik Stockhausen GmbH**
**47805 Krefeld (DE)**

(72) Erfinder:
- **Bruhn, Christoph**
  **47447 Moers (DE)**
- **Herrmann, Edgar, Dr.**
  **41334 Nettetal (DE)**
- **Issberner, Jörg, Dr.**
  **47877 Willich-Neersen (DE)**
- **Kersten, Dagmar**
  **40668 Meerbusch (DE)**
- **Mertens, Richard, Dr.**
  **47803 Krefeld (DE)**
- **Werner, Georg**
  **47918 Tönisvorst (DE)**

(74) Vertreter: **Lang, Arne et al**
**Evonik Degussa GmbH**
**DG-IPM-PAT**
**Bau 1042 / PB 15**
**Paul-Baumann-Strasse 1**
**45764 Marl (DE)**

(56) Entgegenhaltungen:
**WO-A-94/22501**
**DE-A- 4 440 236**
**DE-A- 19 533 269**

EP 1 375 537 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft Absorptionsmittel, vorzugsweise für Wasser und wässrige Flüssigkeiten, auf Basis wässrige Flüssigkeiten absorbierende Polymerisate, in welchen Cyclodextrine oder Cyclodextrinderivate ionisch, kovalent oder durch mechanische Einschlüsse eingebunden sind.

Bei den kommerziell verfügbaren superabsorbierenden Polymeren handelt es sich im Wesentlichen um vernetzte Polyacrylsäuren, vernetzte Stärke/Acrylsäure-Propfcopolymerisate, vernetzte hydrolysierte Stärke/Acrylnitril-Propfcopolymerisate, vemetztes Poly(maleinsäureanhydrid-co-isobutylen) oder Mischungen verschiedener vorgenannter vernetzter Polymere, bei denen die Carboxylgruppen teilweise mit Natrium- und/oder Kalium-Ionen neutralisiert sind.

Solche Polymere finden ihren Einsatz z.B. in Hygieneartikeln, die Körperflüssigkeiten wie z.B. Urin aufsaugen können oder in Materialien zur Umhüllung von Kabeln. Dort nehmen sie unter Quellung und Ausbildung von Hydrogelen große Mengen an wäßrigen Flüssigkeiten und Körperflüssigkeiten, wie z.B. Urin oder Blut auf. Ferner ist es notwendig, daß die aufgenommene Flüssigkeitsmenge unter dem anwendungstypischen Druck zurückgehalten wird. Im Zuge der technischen Weiterentwicklung der superabsorbierenden Polymere hat sich das Anforderungsprofil an diese Produkte über die Jahre hinweg deutlich verändert. Bisher wurde die Entwicklung von Superabsorbern besonders im Hinblick aufgenommener Flüssigkeitsmenge und der Druckstabilität forciert.
Diese vernetzten Polymerisate auf Basis säuregruppenhaltiger Monomere werden durch die Verwendung eines oder mehrerer Vorvernetzer und einem oder mehrerer Nachvernetzer erhalten, und zeigen bislang nicht erreichte Eigenschaftskombinationen aus hoher Retention, hoher Absorption unter Druck, niedrigen löslichen Anteilen und einer schnellen Flüssigkeitsaufnahme. Eingesetzt in Hygieneartikeln haben diese vernetzen Polymerisate den Vorteil, daß die ausgeschiedenen Flüssigkeiten, einmal von dem Polymerisat aufgesogen, keinen Hautkontakt mehr herstellen können. Hautschädigungen wie z.B. Windeldermatitis können so im Wesentlichen vermieden werden. Der Komfort kann zusätzlich gesteigert werden, indem geruchsbelästigende Verbindungen absorbiert werden.

Laut Römpp Chemie Lexikon "unterliegt der Gehalt an Harn-Inhaltsstoffen physiologischen Schwankungen; manche Substanzen werden auch in tagesperiodisch wechselnden Konzentration ausgeschieden, so daß genauere Angaben über die Zusammensetzung des Harns sich stets auf den sog. 24-Stunden-Harn beziehen. Dieser enthält beim gesunden Erwachsenen z.B. Harnstoff (durchschnittlich 20 g), Harnsäure (0,5 g), Kreatinin (1,2 g), Ammoniak (0,5 g), Aminosäuren (2 g), Proteine (60 mg), reduzierende Substanzen (0,5 g, davon etwa 70 mg D-Glucose od. Harnzucker), Zitronensäure (0,5 g) u.a. org. Säuren sowie einige Vitamine (C, $B^{12}$ u.a.). An anorganischen Ionen liegen vor: $Na^+$ (5,9 g), $K^+$ (2,7 g), $NH_4^+$ (0,8 g), $Ca^{2+}$ (0,5 g), $Mg^{2+}$ (0,4 g), $Cl^-$ (8,9 g), $PO_4^{3-}$ (4,1 g), $SO_4^{2-}$ (2,4 g). Der Trockengehalt liegt zwischen 50 u. 72 g. Als flüchtige Komponenten des Harns wurden u.a. Alkylfurane, Ketone, Lactone, Pyrrol, Allylisothiocyanat und Dimethylsulfon erkannt. Es handelt sich bei den flüchtigen Komponenten meist um Moleküle mit einer Molmasse unter ca. 1000 g/mol, die einen hohen Dampfdruck aufweisen.

Flüchtige Komponenten des Harns wurden u. a. auch von A. Zlatkis et al. (Anal. Chem. Vol. 45, 763ff.) untersucht. Bekannt ist weiterhin, daß nach Verzehr von Spargel die Konzentration von organischen schwefelhaltigen Verbindungen im Humanurin zunimmt (R. H. Waring, Xenobiotika, Vol 17, 1363ff). Bei Patienten, die bestimmten Diäten unterliegen, und allgemein bei Patienten die bestimmte Medikamente einnehmen oder bei älteren Menschen, mit nachlassender Nierenfunktion kann der Harn geruchsbelästigende Inhaltsstoffe mit sich führen. Bei Patienten mit Urin-Inkontinenz werden vermehrt Ureasen ausgeschieden, die den im Harn befindlichen Harnstoff umsetzen und so giftiges Ammoniak freisetzen. Bekannt ist ferner eine krankhafte Veränderungen, die Fisch-Geruch-Syndrom genannt wird. Sie beruht auf vermehrter Ausscheidung von quartären Ammoniumverbindungen.

Bisherige Ansätze, um bei Inkontinenzprodukten eine Geruchsverminderung zu erzielen, beruhen auf einer Reduzierung der freien Ammoniak-Konzentration. Dazu gibt es grundsätzlich zwei Ansätze: Verhinderung der zusätzlichen Ammoniak-Produktion aus Harnstoff-Abbau durch geeignete Urease-Hemmer (A. Norberg et al. Gerontology, 1984, 30, 261 ff) oder durch Protonierung von freiem Ammoniak und Bindung desselben als Ammoniumsalz von Carboxylaten. Nachteilig ist bei diesen Verfahren, daß sich im wesentlichen nur Ammoniak und andere stickstoffhaltige Komponenten kontrollieren lassen; geruchsbelästigende Verbindungen ohne basische Gruppierungen, z.B. Thiole, werden weiterhin in den Dampfraum eintreten können.

Dem Fachmann ist bekannt, daß gewisse Hohlraum-Moleküle, auch endohedrale oder konkave Moleküle genannt, andere, meist kleinere, sogenannte Gastmoleküle aufnehmen können und somit einen Wirt/Gastkomplex bilden. Durch diese Komplexbildung werden die chemischen und physikalischen Eigenschaften des Gast- und des Wirtmoleküls beeinflußt. Zu diesen hohlraumbildenden Molekülen gehören Cyclodextrine.

Cyclodextrine entstehen beim Abbau von Stärke durch Bacillus macerans oder Bacillus circulans unter Einwirkung von Cyclodextringlycosyltransferase. Sie bestehen aus 6, 7, 8 oder 9 zu einem Zyklus $\alpha$-1,4-verknüpften Glucose-Einheiten ($\alpha$, $\beta$ bzw. $\gamma$, Cyclodextrine). Sie sind in der Lage, hydrophobe Gastmoleküle in wechselnden Mengen bis zur Sättigung einzuschließen ("molekulare Verkapselung"), z.B. Gase, Alkohole oder Kohlenwasserstoffe. Die Anwendungen von Cyclodextrinen als Wirtmolekül werden in dem Werk von J. Szejtli (Cyclodextrin Technology, Kluwer Academic Publishers, 1988) umfassend referiert.

Es ist auch bereits bekannt, cyclodextrinhaltige Polymere herzustellen. So werden in EP-A-0 483 380 cyclodextrinhaltige Polymere durch Copolymerisation von Aldehydgruppen tragendenden Cyclodextrinen mit Polyvinylalkohol erhalten.

Aus der US-A-5,360,899 sind vernetzte, wasserquellbare, hydrophile Perlpolymerisate aus Hydroxyalkylcyclodextrinen und Vernetzern vom Typ Epichlorhydrin oder Polyepoxiden bekannt. Da diese Vernetzer ein carcinogenes Potential besitzen, ist der Einsatz solcher Produkte in Hygieneartikel nichf möglich. Diese durch Polymerisation immobiliserten Cyclodextrine werden in chromatographischen Trennsäulen als Füll- und Trennmaterial verwendet.

Aus US-A-5,357,012 sind weiterhin wasserquellbare, hydrophile Perlpolymerisate aus Glycidyl- oder Methacrylatgruppen tragenden. Cyclodextrinen und gegebenenfalls weiteren Comonomeren, wie z. B. Hydroxyethylacrylat, bekannt. Auch diese durch Polymerisation immobilisierten Cyclodextrine werden in chromatographischen Trennsäulen als Füll- und Trennmaterial verwendet.

In DE-A-195 20 989 wird die kovalente Anbindung von reaktiven, mindestens einen stickstoffhaltigen Heterocyclus aufweisenden Cyclodextrinderivaten an Polymere, die mindestens eine nukleophile Gruppe tragen, beschrieben. Polymere, die nach dieser Methode mit Cyclodextrinen verknüpft werden, müssen nukleophile Gruppen, wie OH-, NH-, oder SH-Gruppen aufweisen. Auch werden polymerisierbare Cyclodextrinderivate erwähnt, die nach geeigneter Modifizierung mit anderen Monomeren z.B. ethylenisch ungesättigten Monomeren copolymerisiert werden. In dieser Veröffentlichung wird darauf hingewiesen, daß die Produkte nach den o.g. US-Patentschriften US 5,357,012 und US 5,360,899 den Nachteil aufweisen, daß der Cyclodextrineinbau räumlich nur sehr schwer gesteuert werden kann und daß die im Innern der Polymeren fixierten Cyclodextrine für eine Nutzung nicht mehr zur Verfügung stehen. Der Einsatz der Cyclodextrinderivate aufweisenden Polymere als superabsorbierende Materialien wird nicht erwähnt.

Der Einsatz von Cyclodextrinen in Hygieneprodukten ist aus EP-A-806 195, WO 94/22501 und WO 94/22500 u. a. bekannt. Dabei werden die Cyclodextrine zur Geruchsbindung eingesetzt. Soweit die Cyclodextrine bzw. Cyclodextrinkomplexe mit dem pulverförmigen Absorptionsmittel nicht verbunden sind, kann es bei der Lagerung oder dem Transport des Hygieneartikels zu Entmischungen kommen. Dies führt dazu, daß die Effektivität der Cyclodextrine als Geruchsbindungsmittel durch die Entmischung zwischen Absorptionsmittel und Cyclodextrinen verloren gehen kann.

Um eine bessere Haftung auf den pulverförmigen Absorptionsmitteln zu erlangen, lehrt die WO 94/22501 Cyclodextrin mit Polyethylenglykolen oder anderen linearen Polymeren in der "Schmelze" oder in Lösung zu versetzen und dann auf das pulverförmige Absorptionsmittel aufzusprühen. Dem Fachmann ist jedoch bekannt, daß sich lineare Polymere besonders bevorzugt in den Cyclodextrin-Hohlraum "einfädeln". Dies macht man sich in der Supramolekularen Chemie vorteilhaft zu Nutze, um z.B. Rotaxane oder Catenane herzustellen (vgl. dazu die Schriften US 5538655, G. Wenz, Angew. Chem. 1994, 106, 851). Die linearen Polymere haben typischerweise ein Molgewicht ($M_w$) von über 200. Geeignete Polymere sind z.B. auch Polyethylenglycol (PEG), Polyproylenoxid (PEO) und Polyethylenimin Auf einer linearen Polymerkette lassen sich mehrere Cyclodextrine gleichzeitig auffädeln; Harada et al.(J.Org.Chen, 58, 1993, 7524-28) berichten, dass sich 20 Cyclodextrine auf einem Polyethylenglykol mit einer mittleren Molekularmasse von 2000 g/mol auffädeln lassen. Daher ist das beschriebene Verfahren WO94/22501 besonders nachteilig, weil die die Cyclodextrin-Hohlräume nach dieser Vorbehandlung mit einem Polyethylenglykol nicht mehr quantitativ zur Aufnahme von geruchsbelästigenden Verbindungen zur Verfügung stehen.

Die DE -A-195 33269 offenbart ein Verfahren zur Herstellung von Polymerisaten aus wasserunlöslichen Monomeren und ggf. wasserlöslichen Monomeren durch radikalischen Polymerisation der Monomeren in Wasser als Verdünnungsmittel, wobei die wasserlöslichen Monomeren durch Komplexbildung mit Cyclodextrinen und/oder Cyclodextrinstrukturen enthaltenden Verbindungen in eine wasserlösliche Form überführt und so der Polymerisation in wässrigem Medium zugänglich gemacht werden. Die Cyclodextrinkomponente dient bei diesem Verfahren also lediglich als Hilfsmittel dazu, die eingesetzten wasserunlöslichen Monomeren in eine hydrophile Form zu überführen.

Die DE 44 40 236 offenbart redispergierbare Polymerpulver-Zusammensetzungen, die Homo- oder Copolymerisate ethylenisch ungesättigter Monomere und Cyclodextrine oder Cyclodextrinderivate enthalten und ein Verfahren zu deren Herstellung. Die Cyclodextrine oder Cyclodextrinderivate werden in Wasser oder mit Wasser gutmischbaren Lösemitteln wie Aceton oder Ethanol der Polymerisation zugefügt. Die Aufarbeitung erfolgt bevorzugt mittels Sprüh- oder Gefriertrocknung. Die redispergierbaren Polymerpulver-Zusammensetzungen dienen als Bindemittel in der Bauindustrie die höhere Biegefestigkeiten von Formkörpern ermöglichten.

Der vorliegenden Erfindung lag daher die Aufgabe zugrunde, Polymerisate mit der Fähigkeit zur Adsorption von Wasser oder wässrigen Flüssigkeiten und mit der Fähigkeit zur Bindung von geruchsbelästigenden organischen Verbindungen, wie sie z.B. im Harn oder anderen Ausscheidungsflüssigkeiten des Körpers vorkommen, zur Verfügung zu stellen sowie Verfahren zu deren Herstellung.

Diese sollten die Nachteile des Standes der Technik nicht aufweisen und möglichst gleichmäßige, deutliche Reduzierung der im Anwendungsfall abgegebenen gasförmigen geruchsbelästigenden Verbindungen ermöglichen. Darüber hinaus sollte auch erreicht werden, dass eine weitgehend stabile Verteilung der geruchsbindenden Komponenten mit dem Absorptionsmittel vorliegt, d.h. eine Entmischung vor und während der Anwendung möglichst vermieden wird.

Außerdem sollte die Anbindung der geruchsbindenden Komponente nicht unter Verwendung von carcinogener

oder anderweitig gesundheitsschädlicher Substanzen erfolgen. Desweiteren sollte die Wirksamkeit der geruchsbindenden Komponente im Absorptionsmittel unabhängig davon sein, ob sie sich im Innern des Polymers oder an der Oberfläche befindet.

Erfindungsgemäß wird die Aufgabe durch Bereitstellung von Polymerisaten auf Basis von vernetzten, gegebenenfalls teilneutralisierten Säuregruppen tragenden Monomeren gelöst, die Cyclodextrine und/oder Derivate ionisch oder Kovalent gebunden und/oder darin eingeschlossen enthalten.

Aufgrund der erfindungsgemäßen Anbindung an das vorzugsweise pulverförmige Polymerisat, ist die Cyclodextrinkomponente durch die aufzusaugende Flüssigkeit nur noch in vermindertem Umfang extrahierbar bzw. im trockenen Zustand nur noch vermindert entmischbar. Überraschenderweise zeigt das erfindungsmäße Polymerisat trotz der engen Verknüpfung mit dem vernetzten, Säuregruppen tragenden Absorber eine ausgezeichnete Geruchsbindung, die im Vergleich zu ungebundenem Cyclodextrin noch verstärkt ist. Insbesondere zeigen die absorptionsfähigen Polymerisate auch dann sehr gute Geruchsbindung, wenn das Cyclodextrin im Innern des Absorbers fixiert ist. Dies ist durch eine effektive Verringerung der Gas-Konzentration von geruchsbelästigenden Stoffen nachzuweisen.
Darüber hinaus eignen sich die erfindungsgemäßen Polymerisate hervorragend zur Einlagerung von Wirkstoffen, wobei diese Wirkstoffe im Anwendungsfall gegebenenfalls wieder kontrolliert abgegeben werden können. Die Haltbarkeit empfindlicher Wirkstoffe wird durch die Einlagerung in die erfindungsgemäßen Absorptionsmittel deutlich verbessert.

Erfindungsgemäß eignen sich Cyclodextrine des Typs $\alpha$, $\beta$, $\gamma$ sowie deren Derivate.

Die Cyclodextrine weisen die folgende, wiederkehrende Struktur auf

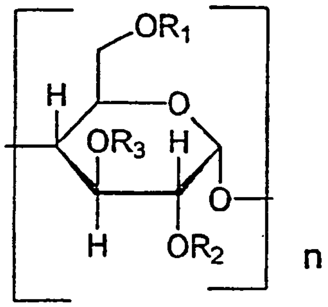

Die Anhydroglykoseeinheiten sind zyklisch zu Ringen glykosionisch verbunden, wobei die Reste $R_1$ bis $R_3$ gleich oder verschieden, für H oder $C_1$-$C_4$-Alkyl und $\alpha$-Cyclodextrin n=6, $\beta$-Cyclodextrin: n = 7, $\gamma$-Cyclodextin: n= 8, $\delta$-Cyclodextrin: n=9 ist.
Bei Cyclodextrinderivaten sind pro Einheit ($R_1$-$R_3$) n verschiede Substituenten möglich, die gleich oder verschieden sein können.

Als Derivate kommen vor allem solche in Betracht, die eine chemische Verknüpfung durch ionische oder kovalente Bindung mit dem Säuregruppen tragenden Monomeren oder den entsprechenden Polymeren ermöglichen. Kovalente Verknüpfungen sind bevorzugt über C-C-Bindungen, wie beispielsweise mit Cyclodextrinderivaten, die ethylenisch ungesättigte Gruppen aufweisen, die bereits bei der Polymerisation der Monomeren kovalent in die Polymerkette eingebunden werden. Solche Gruppen sind beispielsweise (Meth)acryl-, (Meth)allyl- und/oder Vinylgruppe. Andererseits ist es erfindungsgemäß auch möglich, die Cyclodextrinkomponente nach der Polymerisation kovalent durch Ether-, Amid- oder Estergruppen an die Polymere der ethylenisch ungesättigten Monomeren anzuknüpfen.

Die ionische Anbindung der Cyclodextrinderivate kann durch anionische oder kationische Gruppen, wobei die kationischen Gruppen bevorzugt sind, erfolgen. Vielfach ist es von Vorteil, wenn die Cyclodextrinmoleküle mehrfach mit ionischen Gruppen substituiert sind. Beispiele für anionische Gruppen sind Carboxylat-, Sulfat- und Sulfonatgruppen. Beispiele für kationische Gruppen sind quaternäre stickstoffhaltige Gruppen.

Ionische Cyclodextrine können durch Reaktion von Cyclodextrinderivaten mit reaktiven Verbindungen wie z.B. Chloressigsäure, Natriumchloracetat, Maleinsäure, Maleinsäureanhydrid, Bersteinsäureanhydrid hergestellt werden. In wässriger Lösung tragen derartige Umsetungsprodukte z.B. Carboxymethylcyclodextrin im basischen eine negative Ladung durch die Carboxylat-Gruppierung.

Erfindungsgemäß zu verwendende Cyclodextrinderivate mit mindestens einem stickstoffhaltigen Heterocyclus lassen sich der Lehre der DE-A-19520989 herstellen, deren Offenbarung hiermit als Referenz eingeführt wird. Es lassen sich so Cyclodextrinderivate erhalten, die noch eine gegenüber nukleophilen Gruppen aktive Gruppe enthalten. Diese Derivate können direkt mit Polymeren, die ihrerseits nukleophile Gruppen tragen reagieren. Beispiele für nukleophile Gruppen sind: -OH, -NH, oder SH-Gruppen.

Weitere chemisch modifizierte, erfindugsgemäß einzusetzende Cyclodextrinderivate lassen sich erhalten wie in A. P. Croft und R. A. Bartsch, Tetrahedron Vol. 39, No. 9 S.1417-1473 beschrieben. Sie werden erhalten durch

Reaktion von stickstoffhaltigen Verbindungen, die mindestens eine funktionelle Gruppe auweisen, die in der Lage ist, mit den Hydroxylgruppen der Cyclodextrine zu Ether, Ester oder Acetalgruppen zu reagieren.

Besonders bevorzug sind kationische Cyclodextrine wie sie in Ch. Roussel, A. Favrou, Journal of Chromatography A, 704 (1995), 67-74 beschrieben sind. Sie werden erhalten durch Umsetzung von Cyclodextrin mit z.B. N-(3-Chlor-2-hydroxypropyl)-N,N,N-trimethylamoniumchlorid. Die in dieser Veröffentlichung beschriebenen Cyclodextrine haben einen Substitutionsgrad von 0,2.

Die erfindungsgemäß einsetzbaren ionischen Cyclodextrine mit mindestens einem stickstoffhaltigen aliphatischen Rest lassen sich beispielsweise auch nach den in den US 3740391, 4153585 und 4638058 beschriebenen Verfahren herstellen. Die Offenbarung der genannten Vorveröffentlichungen wird hiermit als Referenz eingeführt.

Als geeignete Monomere seien beispielweise genannt: N,N-Dimethylaminoethyl(meth)acrylat, N,N-Dimethylaminopropyl(meth)acrylat, N,N-Dimethylaminoethyl(meth)acrylamid und N,N-Dimethylaminopropyl(meth)acrylamid oder deren durch Umsetzung mit Alkylhalogeniden erhaltenen quarternären Derivate. Bevorzugt Verwendung finden N, N-Dimethylaminoethylacrylat (ADAME bzw. ADAME-quat.) und N,N-Dimethylaminopropylacrylamid (DIMAPA bzw. DIMAPA-quat.).

Dabei erfolgt die Umsetzung mit Verbindung der Formel I:

$$H_2C{=}CR^1\text{-}CO\text{-}X\text{-}R^2\text{-}N(R^3)_3Y^- \qquad (I)$$

in der

$R^1$ = H, $CH_3$

$R^2$ = $C_2$ - $C_4$-Alkylengruppe

$R^3$ = H, $C_1$ - $C_4$-Alkylguppe

X = O, NH

Y = Cl, $SO_4$,

Der durchschnittliche Substitutionsgrad pro Anhydroglukoseeinheit (DS-Wert) läßt sich für stickstoffhaltige. Substituenten nach literaturbekannten Methoden über Elementaranalyse, wie sie beispielsweise in US 5,134,127 und US 3,453,257 für schwefel-bzw. stickstoffhaltige Substituenten beschrieben ist, bestimmen. Mit den in US 3,740,391, 4,153,585 beschriebenen Synthesemethoden läßt sich der DS-Wert in weiten Grenzen variieren.

Pro Anhydroglykoseeinheit eines Cyclodextrins sind 3 Hydroxylgruppen zu weiteren Reaktionen befähigt. Daher kann der Substitutionsgrad z.B. im Falle des β-Cyclodextrins maximal zwischen 0,05 und 3 liegen. Ein Substitutionsgrad unter 0,05 bedeutet, daß eine Mischung aus nicht-modifizierten Cyclodextrin und chemisch modifizierten Cyclodextrin vorliegt.

Erfindungsgemäß beträgt der Substitutionsgrad (DS) der Cyclodextrinderivate 0,005-2, bevorzugt 0,05-1,5.

Ferner können die Cyclodextrine, außer den zuvor erwähnten, für die Anbindung an das Polymerisat erforderliche Gruppen, weitere, dem Polymerisat gegenüber nicht reaktive Substituenten enthalten. Dazu zählen beispielsweise Umsetzungsprodukte von Cyclodextrinen mit Alkylierungsmitteln, wie z. B. $C_1$- bis $C_{22}$-Alkylhalogeniden, z.B. Methylchlorid, Ethylchlorid, Butylchlorid, Butylbromid, Benzylchlorid, Laurylchlorid, Stearylchlorid oder Dimethylsulfat oder Umsetzungsprodukte von Cyclodextrinen mit Alkylenoxiden, wie z. B. Ethylenoxid, Propylenoxid, Butylenoxid oder Styroloxid.

Die erfindungsgemäß einzusetzende Menge an Cyclodextrinen oder deren Derivate, bezogen auf das Gesamtgewicht des Polymerisats, beträgt 0,0110 Gew.%.:

Für die Polymerisation der erfindungsgemäßen Polymerisate, die superabsorbierende Eigenschaften aufweisen können, kommen die bekannten Verfahren in Frage, wie z. B. Massepolymerisation, Lösungspolymerisation, Spraypolymerisation, inverse Emulsionspolymerisation und inverse Suspensionspolymerisation.

Bevorzugt wird die Lösungspolymerisation in Wasser als Lösungsmittel durchgeführt. Die Lösungspolymerisation kann kontinuierlich oder diskontinuierlich erfolgen. Der Stand der Technik weist ein breites Spektrum an Variationsmöglichkeiten hinsichtlich der Konzentrationsverhältnisse, Temperaturen, Art und Menge der Initiatoren als auch der Nachkatalysatoren auf. Typische Verfahren sind in den folgenden Patentschriften beschrieben, die hiermit als Offenbarung des erfindungsgemäßen Verfahrens aufgenommen werden: US 4 286 082, DE 27 06 135, US 4 076 663, DE 35 03 458, DE 40 20 780, DE 42 44 548, DE 43 23 001, DE 43 33 056, DE 44 18 818.

Als Säuregruppen enthaltende, ethylenisch ungesättigte Monomere kommen vorzugsweise aliphatische, gegebenenfalls substituierte $C_2$-$C_{10}$, vorzugsweise $C_2$-$C_5$-Carbonsäuren oder Sulfonsäuren in Frage wie Acrylsäure, Methacrylsäure, Crotonsäure, Isocrotonsäure, Maleinsäure, Furmarsäure, Itaconsäure, Vinylessigsäure, Vinylsulfonsäure, Methallylsulfonsäure, 2-Acrylamido-2-methyl-1-propansulfonsäure sowie deren Alkali- und/oder Ammoniumsalze oder deren Gemische vor. Bevorzugt wird Acrylsäure und deren Gemische sowie deren Alkali- und/oder Ammoniumsalze verwendet. Ferner ist es auch möglich, Monomere zu verwenden, die erst nach der Polymerisation zu Säuregruppen hydrolisiert werden, wie z. B.die entsprechenden Nitrilverbindungen.

Zur Modifizierung der Polymereigenschaften können gegebenenfalls bis zu 40 Gew.% weitere, von den säuregruppenhaltigen Monomeren unterschiedliche Monomere, die im wässrigen Polymerisationsansatz löslich sind, wie beispielsweise Acrylamid, Methacrylamid, Acrylnitril, (Meth)allylalkoholethoxylate und die Mono(Meth)acrylsäureester von mehrwertigen Alkoholen oder Ethoxylaten, verwendet werden.

Zusammen mit den o.g. Monomeren werden in geringen Anteilen vernetzend wirkende Monomere mit mehr als einer reaktionsfähigen Gruppe im Molekül mitpolymerisiert. Dabei entstehen teilvernetzte Polymerisate, die nicht mehr in Wasser löslich, sondern nur mehr quellbar sind. Als vernetzende Monomere seien beispielsweise bi- oder höherfunktionelle Monomere, wie z. B.Methylenbisacryl- bzw. -methacrylamid oder Ethylenbisacrylamid, genannt, ferner Allylverbindungen wie Allyl(meth)acrylat, alkoxyliertes Allyl(meth)acrylat mit vorzugsweise 1 bis 30 Mol Ethylenoxideinheiten Triallylcyanurat, Maleinsäurediallylester, Polyallylester, Tetraallyloxiethan, Triallylamin, Tetraallylethylendiamin, Allylester der Phosphorsäure bzw. phosphorigen Säure, ferner die N-Methylolverbindungen von ungesättigten Amiden, wie z. B. von Methacrylamid bzw. Acrylamid und die abgeleiteten Ether sowie Ester von Polyolen und alkoxylierten Polyolen mit ungesättigten Säuren, wie Diacrylate oder Triacrylate z.B. Butandiol- oder Ethylenglykoldiacrylat, Polyglykol-di-(meth)acrylate, Trimethylolpropantriacrylat, Di- und Triacrylatester des, vorzugsweise mit 1 bis 30 Mol Alkylenoxid oxalkylierten (ethoxylierten) Trimethylolpropans, Acrylat- und Methacrylatester von Glycerin und Pentaerythrit, sowie des mit vorzugsweise 1 bis 30 Mol Ethylenoxid oxethylierten Glycerins und Pentaerythrits. Bevorzugt werden Triallylamin, Acrylate mehrwertiger Alkohole bzw. deren Alkoxylate und Methallylalkoholacrylate bzw. deren Alkoxylate verwendet. Der Anteil an den vernetzenden Comonomeren liegt bei 0,01 bis 3,0 Gew%, bevorzugt bei 0,05 bis 2,0 Gew% und besonders bevorzugt bei 0,05 bis 1,5 Gew.%, bezogen auf das Gesamtgewicht der Monomeren.

Die gegebenenfalls vorgenommene Neutralisation der sauren Monomeren nach dem erfindungsgemäßen Polymerisationsverfahren kann auf verschiedene Art und Weise durchgeführt werden. Zum einen kann die Polymerisation entsprechend der Lehre der US 4 654 039 direkt mit den sauren Monomeren durchgeführt werden, wobei die Neutralisation dann anschließend im Polymergel erfolgt. Bevorzugt werden die Säuregruppen der Monomere bereits vor der Polymerisation zu 20-95%, bevorzugt 50-80% neutralisiert und liegen dann bereits bei Beginn der Polymerisation als Natrium-, und/oder Kalium und/oder Ammoniumsalze vor. Zur Neutralisation werden bevorzugt solche Basen genommen, die keinen negativen Einfluß auf die spätere Polymerisation haben. Bevorzugt wird Natron- und/oder Kalilauge und oder Ammoniak, besonders bevorzugt Natronlauge, genommen, wobei ein Zusatz von Natriumcarbonat, Kaliumcarbonat oder Natriumbicarbonat einen zusätzlichen positiven Effekt gemäß der Lehre der US 5 314 420 und der US 5 154 713 ausüben kann. Diese teilneutralisierte Monomerlösung wird vor dem Start der Polymerisation bei der adiabatischen Lösungspolymerisation auf eine Temperatur von unter 30°C, bevorzugt unter 20°C gekühlt. Bei den anderen Polymerisationsverfahren werden die aus dem Stand der Technik bekannten Temperaturen eingehalten, wie sie aus der nachstehenden Literatur hervorgehen.

Die erfindungsgemäßen Polymerisate können gegebenenfalls wasserlösliche natürliche oder synthetische Polymere als Pfropfgrundlage in Mengen bis zu 30 Gew.% enthalten. Dazu zählen unter anderem teil- oder vollverseifte Polyvinylalkohole, Stärke oder Stärkederivate, Cellulose oder Cellulosederivate, Polyacrylsäuren, Polyglykole oder deren Gemische. Die Molekulargewichte der als Pfropfgrundlage zugesetzten Polymere müssen an die Gegebenheiten der Polymerisationsbedingungen angepaßt sein. So kann es z.B. im Falle einer wässrigen Lösungspolymerisation aus Gründen der Viskosität der Polymerisatlösung erforderlich sein, Polymere mit niedrigen bis mittleren Molekulargewichten einzusetzen, wohingegen bei der Suspensionspolymerisation dieser Faktor eine untergeordnete Rolle spielt.

Neben Polymerisaten, die durch vernetzende Polymerisation teilneutralisierter Acrylsäure erhalten werden, werden bevorzugt solche verwendet, die durch Einsatz von Stärke oder Polyvinylalkohol als Pfropfgrundlage enthalten werden.

Das erfindungsgemäße Polymerisationsverfahren kann durch verschiedene Bedingungen initiert werden, wie z.B. durch Bestrahlung mit radioaktiven, elektromagnetischen oder ultravioletten Strahlen oder durch Redoxreaktion zweier Verbindungen, wie z.B. Natriumhydrogensulfit mit Kaliumpersulfat oder Ascorbinsäure mit Wasserstoffperoxid. Auch der thermisch ausgelöste Zerfall eines sogenannten Radikalstarters wie beispielsweise Azobisisobutyronitril, Natriumperoxidisulfat, t-Butylhydroperoxid oder Dibenzoylperoxid ist geeignet. Ferner ist die Kombination von mehreren der genannten Starter bzw. Polymerisationsinitiatoren möglich.

Die Herstellung der erfindungsgemäßen Polymerisate erfolgt vorzugsweise nach zwei Methoden:

Nach der ersten Methode wird die teilneutralisierte Acrylsäure in wäßriger Lösung in Gegenwart der Vemetzer und gegebenenfalls der Polymerzusätze durch radikalische Polymerisation in ein Gel überführt, das dann zerkleinert und bis zum Erreichen eines pulverförrrügen, rieselfähigen Zustandes getrocknet und gemahlen und auf die gewünschte Partikelgröße abgesiebt wird. Die Lösungspolymerisation kann kontinuierlich oder diskontinuierlich durchgeführt werden.

Die Patentliteratur weist sowohl ein breites Spektrum an Variationsmöglichkeiten hinsichtlich der Konzentrationsverhältnisse, Temperaturen, Art und Menge der Initiatoren als auch eine Vielzahl von Nachvernetzungsmöglichkeiten aus. Typische Verfahren sind in den folgenden Patentschriften beschrieben, deren Offenbarung hiermit als Referenz eingeführt wird:
US 4 076 663, US 4 286 082, DE 27 06 135, DE 35 03 458, DE 35 44 770, DE 40 20 780, DE 42 44 548, DE 43 23 001, DE 43 33 056, DE 44 18 818.

Auch das inverse Suspensions- und Emulsionspolymerisationsverfahren kann zur Herstellung der erfindungsgemäßen Polymerisate angewendet werden. Nach dieser Verfahrensvariante wird eine wässrige, teilneutralisierte Acrylsäurelösung mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen vorgelegt, oder aber separat und gegebenfalls während der Polymerisation zugefügt. Die Zugabe von gegebenenfalls vorhandenen polymeren Pfropfgrundlagen erfolgt über die Monomerlösung oder durch direkte Vorlage in die Ölphase. Anschließend wird das Wasser azeotrop aus dem Gemisch entfernt und das Polymerprodukt abfiltriert sowie optional getrocknet.

Die erfindungsgemäßen Polymerisate werden durch eine nachträgliche Oberflächenvernetzung in ihrem Eigenschaftsprofil verbessert, insbesondere auch in ihrer Flüssigkeitsaufnahme unter Druck, damit das bekannte Phänomen des "gel blocking" unterdrückt wird, bei dem angequollenen Polymerteilchen miteinander verkleben und eine weitere Flüssigkeitsaufnahme und Flüssigkeitsverteilung in den Absorptionsartikeln behindern. Während der Nachvernetzung werden die Carboxylgruppen der Polymermoleküle an der Oberfläche der Polymerisatteilchen mit Vernetzungsmitteln unter erhöhter Temperatur vernetzt. Verfahren zur Nachvernetzung sind u. a. in den nachstehenden Veröffentlichungen angegeben, wie z. B. in DE 40 20 780 , in EP 317 106 und in WO 94/9043. Die dem Fachmann z. B aus der US 5 314 420, Seite 8, Zeile 3-45 bekannten Oberflächenvernetzungsmittel können erfindungsgemäß alle vorteilhaft in Kombination mit einem während der Polymerisation verwendeten Vernetzer oder einer Kombination von Vernetzern eingesetzt werden. Diese Verbindungen enthalten in der Regel mindestens zwei funktionelle, Gruppen, die zur Reaktion mit Carbonsäure- oder Carboxylgruppen befähigt sind. Dabei sind Alkohol-, Amin-, Aldehyd- und Carbonatgruppen bevorzugt, wobei auch Vernetzermoleküle mit mehreren verschiedenen Funktionen eingesetzt werden. Bevorzugt werden Polyole, Polyamine, Polyaminoalkohole, und Alkylencarbonate eingesetzt. Bevorzugt wird eines der folgenden Vernetzungsmittel eingesetzt: Ethylenglykol, Diethylenglykol, Triethylenglykol, Polyethylenglykol, Glycerin, Polyglycerin, Propylenglykol, Diethanolamin, Triethanolamin, Polypropylenglykol, Blockcopolymere aus Ethylenoxyd und Propylenoxyd, Sorbitanfettsäureester, ethoxylierte Sorbitanfettsäureester, Trimethylolpropan, ethoxyliertes Trimethylolpropan, Pentaerythrit, ethoxyliertes Pantaerythrit, Polyvinylalkol, Sorbit, Ethylencarbonat, Proypylencarbonat. Besonders bevorzugt wird mit Polyolen und Ethylencarbonat als Oberflächenvernetzungsmittel gearbeitet. Das Vernetzungsmittel wird vorzugsweise in einer Menge von 0,01 bis 30 Gewichtsprozent, bevorzugt 0,1-10 Gewichtsprozent, bezogen auf das zu vernetzende Polymerisat, eingesetzt.

Das Polymerisat wird nach der Polymerisation getrocknet, gemahlen und auf die für die jeweils anwendungstechnisch günstige Kornfraktion abgesiebt und dann der Oberflächenvernetzung zugeführt. In manchen Fällen hat es sich jedoch auch bewährt, die Oberflächen-Nachvernetzer bereits vor der Trocknung des Polymergels bzw. vor der Zerkleinerung des teilweise oder überwiegend getrockneten Polymerisats zuzufügen. Eine erfindungsgemäß durchzuführende Nachvernetzung wird z. B. in der US 4 666 983 und der DE 40 20 780 beschrieben, die als Referenz eingeführt werden. Der Zusatz der Nachvernetzer erfolgt häufig vorteilhafterweise auch in Form einer Lösung in Wasser, organischen Lösemitteln oder deren Mischungen, insbesondere dann, wenn geringe Mengen an Nachvernetzungsmittel angewandt werden. Geeignete Mischaggregate zum Aufbringen des Nachvernetzungsmittels sind z.B. Patterson-Kelley-Mischer, DRAIS-Turbulenzmischer, Lödigemischer, Ruberg-Mischer, Schneckenmischer, Tellermischer und Wirbelschichtmischer, sowie kontinuierlich arbeitende senkrechte Mischer in denen das Pulver mittels rotierender Messer in schneller Frequenz gemischt wird (Schugi-Mischer). Nachdem der Oberflächenvernetzer mit dem vernetzten Polymerisat vermischt worden ist, wird zur Durchführung der Oberflächenvernetzungsreaktion auf Temperaturen von 60 bis 250 °C, bevorzugt auf 135 bis 200°C und besonders bevorzugt auf 150 bis 185°C erhitzt. Die Zeitdauer der Wärmebehandlung wird durch die Gefahr, daß das gewünschte Eigenschaftsprofil des superabsorbierenden Polymerisats infolge von Hitzeeinwirkung zerstört wird, begrenzt.

Für die Verarbeitung der Polymerisate als Superabsorber werden je nach Anwendungsfall unterschiedliche Siebfraktionen eingesetzt, so z.B. für Windeln zwischen 100 und 1000 μm, bevorzugt zwischen 150 und 850 μm. Diese Kornfraktion wird im allgemeinen durch Mahlung und Siebung vor und oder nach der Nachvernetzung hergestellt.

Gemäß dem erfindungsgemäßen Verfahren werden die Cyclodextrine oder deren Derivate in Substanz, oder in einem Lösungsmittel gelöst, angewendet. Ein bevorzugtes Lösemittel ist Wasser, jedoch kommen auch Gemische aus Wasser und organische Lösemitteln zum Einsatz, wie Ethylalkohol, Azeton.

Die Zugabe der Cyclodextrinkomponente kann in verschiedenen Verfahrensstufen der Herstellung des erfindungsgemäßen Polymerisate erfolgen. Die Menge an Cyclodextrinen oder deren Derivaten, bezogen auf die Menge Polymerisat, beträgt 0,01-10 Gew.%.:

So kann die Zugabe zur Monomerlösung erfolgen. Dabei wird Cyclodextrin oder dessen Derivat direkt in die wässrige Monomerlösung vor deren Polymerisation zugegeben. Wird das erfindungsgemäße Polymerisat durch Suspensionspolymerisation hergestellt, so ist es auch möglich, daß Cyclodextrin in der Ölphase ganz oder teilweise vorzulegen und die Monomerlösung dazu zu dosieren. Wird nur ein Teil das Cyclodextrins vorgelegt, so kann der Rest über die Monomerlösung eingetragen werden.

Es ist auch möglich, die Cyclodextrinkomponente auf das nicht getrocknete Polymergel aufzubringen. Dabei wird Cyclodextrin oder dessen Derivat in Substanz oder gelöst in Wasser und/oder einem organischen Lösungsmittel auf das zerkleinerte Polymergel aufgebracht, vorzugsweise durch Aufsprühren und Vermischen.

Es ist jedoch auch möglich, das Polymergel zunächst zu trocknen, zu zerkleinern und anschließend Cyclodextrin oder dessen Derivat in Substanz oder gelöst in Wasser und/oder einem organischen Lösungsmittel auf das Pulver aufzutragen. Das resultierende Produkt kann direkt weiterverarbeitet oder getrocknet werden, um Lösungsmittel zu entfernen.

Es ist auch möglich, die Cyclodextrinkomponente auf das zerkleinerte und getrocknete Absorptionsmaterial während der Oberflächenvernetzung des Polymerisats zuzugeben. Geeignete Mischaggregate zum Aufbringen des Vernetzungsmittels und der Cyclodextrinkomponente sind z. B. Patterson-Kelley-Mischer, DRAIS-Turbulenzmischer, Lödigemischer, Ruberg-Mischer, Schneckenmischer, Tellermischer und Wirbelschichtmischer, sowie kontinuierlich arbeitende senkrechte Mischer in denen das Pulver mittels rotierender Messer in schneller Frequenz gemischt wird (Schugi-Mischer).

Es ist auch möglich, die Cyclodextrinkomponente auf das zerkleinerte, bereits oberflächenvernetzte Polymerisat aufzubringen. Für diese Verfahrensvariante werden erfindungsgemäß, vorzugsweise ionisch modifizierte Cyclodextrine in Substanz oder gelöst in Wasser und/oder einem organischen Lösungsmittel auf das vorzugsweise pulverförmige Polymerisat aufgesprüht und anschließend das Lösungsmittel verdampft.

Nach dem erfindungsgemäßen Verfahren ist es aber auch möglich, die Cyclodextrinkomponente in verschiedenen Stufen des Herstellungsprozesses einzubringen, um deren Wirkung gegebenenfalls zu optimieren. Dadurch ist es beispielsweise möglich, ein nicht modifiziertes Cyclodextrin bereits mit der Monomerlösung zu polymerisierten und im Zuge der Oberflächenvernetzung des Polymerisats ein ionisch modifiziertes Cyclodextrin an der Oberfläche des Polymerisats zu fixieren.

Es ist auch möglich, die Cyclodextrinkomponente bei einer weiteren Oberflächenvernetzung mit dem Polymerisat zu verbinden.

Es werden nach den erfindungsgemäßen Verfahren Endprodukte erhalten, in denen das Cyclodextrin oder dessen Derivat im synthetischen Polymer so eingebunden ist, daß die mit Wasser extrahierbare Menge an Cyclodextrin deutlich geringer ist als die tatsächlich im Endprodukt enthaltende Menge. Der extrahierbare Anteil an Cyclodextrinen unterschreitet bei den erfindungsgemäßen Produkten 85% der im Produkt vorhandenden Menge, bevorzugt 60%, besonders bevorzugt 45%.

Die erfindungsgemäßen Polymerisate eignen sich aufgrund ihrer ausgezeichneten Absorptionsfähigkeit als Absorptionsmittel, die im Vergleich zu pulverförmigen Absorptionsmitteln ohne Cyclodextrin oder dessen Derivat, eine bessere Aufnahme von geruchsbelästigenden Verbindungen aufweisen.

Die erfindungsgemäßen Polymere finden ihren Einsatz z. B. in Hygieneartikeln, die Körperflüssigkeiten, wie z. B. Urin aufsaugen können, oder in Verpackungsbereich von z. B. Fleisch und Fischprodukten. Dort nehmen sie unter Quellung und Ausbildung von Hydrogelen große Mengen an wäßrigen Flüssigkeiten und Körperflüssigkeiten, wie z.B. Urin oder Blut auf. Die erfindungsgemäßen Polymerisate werden direkt als Pulver in die Konstruktionen zur Aufnahme von Flüssigkeiten eingearbeitet, oder vorher in geschäumten oder nicht geschäumten Flächengebilden fixiert. Derartige Konstruktionen zur Aufnahme von Flüssigkeiten sind beispielsweise Baby Windeln, Inkontinenzprodukte, saugfähige Einlagen in Verpackungseinheiten für Lebensmittel.

Darüber hinaus hat es sich gezeigt, daß sich die erfindungsgemäßen Absorptionsmittel hervorragend zur Einlagerung von Wirkstoffen geeignet sind. Die Haltbarkeit empfindlicher Wirkstoffe, beispielsweise gegen oxidativen Abbau, wird durch die Einlagerung in die erfindungsgemäßen Absorptionsmittel wesentlich verbessert.

Weiterhin finden die erfindungsgemäßen Polymere Anwendung bei der Pflanzenaufzucht und bei der Schädlingsbekämpfung in der Landwirtschaft. In der Pflanzenaufzucht sorgen die Polymere in der Nähe von Pflanzenwurzel für eine ausreichende Zufuhr von Flüssigkeit und von zuvor eingelagerten Nährstoffen und sind in der Lage diese über einen längeren Zeitraum zu speichern und wieder freizusetzen.

Bei der Schädlingsbekämpfung lassen sich in den erfindungsgemäßen Polymeren Wirkstoffe einzeln oder in Kombination von mehreren Wirkstoffen einlagern, die dann im Anwendungsfall Zeit und Mengenkontrolliert freigesetzt werden.

In den folgenden Beispielen, die auch die Herstellung erfindungsgemäß verwendeter ionischer Cyclodextrine umfassen, werden die Herstellung und Eigenschaften der erfindungsgemäßen Polymerisate erläutert.

Prüfmethoden der erfindungsgemäßen Polymerisate:

1) 1 g pulverförmiges Polymerisat wird mit 180 ml einer wässrigen Kochsalzlösung übergossen und 1h (alternativ 16h) bei Raumtemperatur gut gerührt. Anschließend wird durch ein Sieb filtriert und die Konzentration Cyclodextrin gemäß nachfolgeneder Methode bestimmt.
Die Methode beruht auf der Abnahme der Licht-Absorption (550 nm) einer alkalischen Phenolphthaleinlösung in Gegenwart von Cyclodextrin und kann wie von T. Takeuchi und T. Miwa, Chromatographia 1994, 38, 453, beschrieben, bestimmt werden.
Es wird die experimentell erhaltene Konzentration durch die theoretisch berechnete Konzentration dividiert. Die theoretische Konzentration läßt sich ermitteln aus der eingesetzten Menge Cyclodextrin in dem pulverförmigen Absorptionsmittel dividiert durch180. Man erhält so den extrahierten Anteil an Cyclodextrin.

$$EA(CD) = \frac{\text{gefundene Konzentration (CD)}}{\text{theoretische Konzentration (CD)}}$$

EA(CD) Extrahierbarer Anteil von Cyclodextrin

2) Bestimmung der Aufnahme von geruchsbelästigenden Verbindungen
0, 1 g pulverförmiges Polymerisat werden mit 2 ml einer wässrigen Lösung (enthält 5 Gew.% Ethanol) der geruchsbelästigenden Verbindung versetzt und in einem 5 ml-fassenden Probengefäß verschlossen. Man läßt bei 40°C für 20 min stehen und untersucht per Head-Space GC quantitativ den Gehalt der geruchsbelästigenden Verbindung im Dampfraum über der Flüssigkeit gegen eine Nullprobe.

Beispiele:

Vergleichsbeispiel 1) nach Patentanmeldung WO 94/22500 bwz. WO 94/22501 9,850 g eines handelsüblichen pulverförmigen Absorptionsmittels (Favor®, Firma Stockhausen GmbH) werden mit 0,15 g festes β-Cyclodextrin (beta-W7-Cyclodextrin technisch, der Firma Wacker) gut durchmischt. Anschließend wird nach der angegebenen Prüfmethode der extrahierbare Anteil an Cyclodextrin bestimmt.
EA = 93 %

Vergleichsbeispiel 2) nach Patentanmeldung WO 94/22500 bzw. WO 94/22501 40 g Polyethylenglykol (Mw 3000) werden bei erhöhter Temperatur geschmolzen. Hierzu gibt man 40 g Cyclodextrin und homogenisiert die Mischung. 9,40 g eines handelsüblichen pulverförmigen Absorptionsmittels (Favor®, Firma Stockhausen GmbH) werden mit 0,6 g der Cyclodextrin/Polyethenlenglycol-Lösung besprüht gut durchmischt und auf Raumtemperatur gekühlt. Anschließend wird nach der angegebenen Prüfmethode der extrahierbare Anteil an Cyclodextrin bestimmt.
EA=89%

Beispiel 1)

A) Eine wässrige Acrylsäure - Lösung (29,3 Gew.%) wird mit 1,2 Gew.%/Monomer eines Polyglykolacrylat-Vernetzergemisches vermischt und unter Rühren und Kühlen mit 50%-iger Natronlauge zu 60 Mol% teilneutralisiert. Die Lösung wird auf 7-8°C abgekühlt und mit Stickstoff für ca. 20 min. durchperlt. Die Polymerisation wird nach Zugabe von wäßrigen Lösungen von Natriumpersulfat, Wasserstoffperoxid und einem wasserlöslichen Azoinitiator mit Ascorbinsäure gestartet, worauf eine deutliche Temperaturerhöhung auf über 90°C stattfindet. Man erhält ein gelförmiges Produkt.

B) 50 g des getrockneten, gemahlenen und auf 150-800 mm abgesiebten Polymeren aus A) werden unter kräftigen Durchmischen mit einer Lösung aus 0,5 g Ethylencarbonat, 2 g Wasser und 4 g Aceton in einem Kunststoffgefäß benetzt und mit einem handelsüblichen Haushalts-Handmixgerät (Firma Krupps) gut durchmischt. Anschließend wird das benetzte Polymer für 30 Minuten in einem Ofen auf eine Temperatur von 180°C erhitzt, wobei es oberflächenvernetzt wird.

C) Man verfährt wie unter A) beschrieben. Zusätzlich werden der Monomerlösung aber 5g Cyclodextrin beigefügt. Man erhält ein gelförmiges Produkt, dessen Weiterverarbeitung wie unter B) beschrieben erfolgt.

D) Das aus A) erhaltene cyclodextrinfreie Gel wird in einem Becherglas in eine 80°C heiße Lösung bestehend aus 10 g Cyclodextrin und 23,3 g Wasser getaucht, bis die Lösung in das Polymergel vollständig eingedrungen ist. Anschließend wird das Gel gewölft und bei 150°C getrocknet.
EA = 27 %, bestimmt nach der angegebenen Prüfmethode

E) 50 g des getrockneten, gemahlenen und auf 150-800 μm abgesiebten Polymeren aus D) werden unter kräftigen Durchmischen mit einer Lösung aus 0,5 g Ethylencarbonat, 2 g Wasser und 4 g Aceton in einem Kunststoffgefäß benetzt und mit einem handelsüblichen Haushalts-Handmixgerät (Firma Krupps) gut durchmischt. Anschließend wird das benetzte Polymer für 30 Minuten in einem Ofen auf eine Temperatur von 180°C erhitzt.

Der extrahierbare Anteil mit EA = 8 %, bestimmt nach der angegebenen Prüfmethode ist deutlich niedriger durch die Oberflächenvemetzung.

Beispiel 2)

50 g des gewölften, getrockneten, gemahlenen und auf 150-800 μm abgesiebten Polymeren aus Beispiel 1 A) werden unter kräftigen Durchmischen mit einer Lösung aus 0,5 g Ethylencarbonat, 1,5 g nicht modifiziertes Cyclodextrin und 8,5 g Wasser in einem Kunststoffgefäß benetzt und mit einem handelsüblichen Haushalts-Handmixgerät (Firma Krupps) gut durchmischt. Anschließend wird das benetzte Polymer für 25 Minuten in einem Ofen zur Oberfiächenvernetzung auf eine Temperatur von 175°C erhitzt.
EA = 80%, bestimmt nach der angegebenen Prüfmethode

Beispiel 3)

F) In einem 500ml Dreihals Rundkolben werden 113,4 g β-Cyclodextin in 200 g entionisiertes Wasser und 8 g einer wässrigen Natronlauge (50%-ig) suspendiert. Man erwärmt diese Suspension bis zum Sieden, bis alles in Lösung gegangen ist. Unter kräftigem Rühren werden nun 34,4 g einer wässrigen Lösung DIMAPA-quat. (60%-ig) innerhalb 30 min zugetropft und für weitere 5h unter Rückfluß gerührt. Man kühlt die Lösung auf 5°C ab und stellt mit Salzsäure einen pH von 7 ein. Der Niederschlag wird filtriert und mit Wasser gewaschen. Nach dem Trocknen des Filterrückstandes wird der DS-Wert per Elementaranalyse auf 0,005 ermittelt. 50 g des gewölften, getrockneten, gemahlenen, nachvernetzten und auf 150-800 μm abgesiebten Polymeren aus Beispiel 1 B) werden unter kräftigen Durchmischen mit einer Lösung aus 0,5 g Ethylencarbonat, 1,5 g Cyclodextrinderivat gemäß F) und 7,3 g Wasser in einem Kunststoffgefäß benetzt und mit einem handelsüblichen Haushalts-Handmixgerät (Firma Krupps) gut durchmischt. Anschließend wird das benetzte Polymer für 25 Minuten in einem Ofen auf eine Temperatur von 175°C erhitzt, um es oberflächenzuvernetzen.

EA = 40%, bestimmt nach der angegebenen Prüfmethode.
Bestimmung der Gaskonzentration geruchsbelästigender Verbindungen.
Oberflächennachvernetzte Superabsorber aus vemetzter Polyacrylsäure mit Neutralisationsgraden von 60 bzw. 70 % wurden im Zuge einer zweiten Oberflächennachvernetzung mit unterschiedlichen Cyclodextrinen gemäß dem Verfahren nach Beispiel 3 modifiziert. Die Menge an Cyclodextrin ergibt sich aus nachfolgender Tabelle. Bei der Messung der geruchsbelästigenden Stoffe wurde als Blindprobe gemäß der angegebenen Prüfvorschrift ein Polymer ohne Cyclodextrin untersucht und die gefundene Gaskonzentration der geruchsbelästigenden Substanz mit 100% gleichgesetzt. Anschließend wurden Cyclodextrin-haltige Proben untersucht und die Gaskonzentration der geruchsbelästigenden Substanz bestimmt.
Geruchsstoff: Ethylfuran:

| Gew. %-Anteil CD | Cyclodextrin-Derivat | Abnahme der Konzentration von Ethylfuran im Gasraum |
|---|---|---|
| 10## | β-Cyclodextrin | 72% |
| 3## | β-Cyclodextrin | 63% |
| 3## | α-Cyclodextrin | 68% |
| ##: Absorber mit 60%iger Neutralisation der Säuregruppen | | |

Es ist klar zu erkennen, daß die Gaskonzentration von flüchtigen, in Wasser gelösten Substanzen nach Absorption durch die erfindungsgemäßen, Cyclodextrin enthaltenden Polymere abnehmen.

Analog zum Ethylfuran wurde ein schwefelhaltiger Geruchsstoff untersucht.

Zusätzlich wurde der Effekt von reinem Cyclodextrin (ohne Polymer) verfolgt. Man erkennt, daß schon ab einem Gehalt von 3% Cyclodextrin im erfindungsgemäßen Polymer eine deutliche Reduzierung der Gaskonzentration der schwefelhaltigen Verbindung erreicht werden kann.

Dotierung mit Fufurylmercaptan;

| Gew. %-Anteil CD | Cyclodextrin bzw. CD-Derivat | Abnahme der Konzentration von Fufurylmercaptan im Gasraum |
|---|---|---|
| 10# | β-Cyclodextrin | 42% |
| 3# | β-Cyclodextrin | 51% |
| 3# | α-Cyclodextrin | 65% |
| 10## | β-Cyclodextrin | 46% |
| 3## | β-Cyclodextrin | 18% |
| 3## | α-Cyclodextrin | 28% |
| 1,5# | Monochlortriazinyl β-Cyclodextrin | 42% |
| 3# | Monochlortriazinyl β-Cyclodextrin | 49% |
| 100 | β-Cyclodextrin | 57% |
| 100 | α-Cyclodextrin | 64% |

#: Absorber mit 70%iger Neutralisation der Säuregruppen
##: Absorber mit 60%iger Neutralisation der Säuregruppen

Ausgezeichnete geruchsbindende Wirkung entfalten die erfindungsgemäßen Polymere , wenn das Cyclodextrin in den Polymeren eingeschlossen ist:

| Polymer gemäß Beispiel | CD-Anteil [Gew.%] | CD-Derivat | Abnahme der Konz. von Furfurylmercaptan im Gasraum [%] |
|---|---|---|---|
| 1 E | 1,5 | nicht modifiz. | 72 |
| 1 E | 3 | nicht modifiz. | 55 |
| 3 | 3 | nach Bsp.3 F | 49 |

## Patentansprüche

1. Absorptionsfähiges Polymerisat auf Basis von gegebenenfalls teilneutralisierten, monoethylenisch ungesättigten, säuregruppentragenden Monomeren, dessen Oberfläche nach der Polymerisation nachvernetzt wurde, erhältlich durch ein Verfahren, beim dem

    - eine wässrige Lösung eines ethylenisch ungesättigten Säuregruppentragendell, gegebenenfalls teilneutralisierten Monomeren und eines vernetzenden Monomeren radikalisch polymerisiert wird,
    - das Polymerisat anschließend getrocknet und gemahlen wird,
    - das Polymerisat vor oder nach der Trocknung oberflächenvenietzt wird,

    **dadurch gekennzeichnet, dass** Cyclodextrine oder deren Derivate in Substanz oder in einem Lösungsmittel gelöst

    - der Monomerlösung vor deren Polymerisation zugesetzt werden, oder
    - auf das nicht getrocknete Polymergel aufgebracht werden, oder
    - auf das getrocknete und zerkleinerte Polymergel aufgetragen werden, wobei die Cyclodextrine oder deren Derivate gegebenenfalls während der Oberflächenvernetzung zugegeben werden, oder
    - auf das zerkleinerte und bereits oberflächenvemetzte Polymerisat aufgebracht werden.

2. Absorptionsfähiges Polymerisat nach Anspruch 1, **dadurch gekennzeichnet, dass** der extrahierbare Anteil an Cyclodextrinen kleiner als 85 % der im Produkt vorhandenen Menge ist.

3. Absorptionsfähiges Polymerisat nach Anspruch 1 oder 2, **dadurch gekennzeichnet, dass** der extrahierbare Anteil

an Cyclodextrinen kleiner als 60 % der im Produkt vorhandenen Menge ist.

4. Absorptionsfähiges Polymerisat nach einem der vorhergehenden Ansprüche, **dadurch gekennzeichnet, dass** der extrahierbare Anteil an Cyclodextrinen kleiner als 45 % der im Produkt vorhandenen Menge ist.

5. Absorptionsfähiges Polymerisat nach einem der vorhergehenden Ansprüche, **dadurch gekennzeichnet, dass dadurch, dass** die Menge an Cyclodextrinen oder Cyclodextrinderivaten 0,01 bis 10 Gew.%, bezogen auf die Menge Polymerisat, beträgt.

6. Absorptionsfähiges Polymerisat nach einem der vorhergehenden Ansprüche, **dadurch gekennzeichnet, dass** es sich bei den Cyclodextrinen um α-, β- oder γ-Cyclodextrine handelt.

7. Absorptionsfähiges Polymerisat nach einem der vorhergehenden Ansprüche, **dadurch gekennzeichnet dass** die Cyclodextrine oder Cyclodextrinderivate kovalent oder ionisch mit dem Polymer gebunden sind.

8. Konstruktion zur Aufnahme von Flüssigkeiten, beinhaltend ein absorptionsfähiges Polymerisat nach einem der vorhergehenden Ansprüche.

9. Konstruktion nach Anspruch 8, **dadurch gekennzeichnet**, **dadurch gekennzeichnet, dass** die Konstruktion eine Windel oder ein Inkontinenzprodukt ist,

10. Ein Verfahren zur Herstellung eines absorptionsfähigen Polymerisates auf Basis von gegebenenfalls teilneutralisierten, monoethylenisch ungesättigten, säuregruppentragenden Monomeren, dessen Oberfläche nach der Polymerisation nachvernetzt wurde, beim dem

   - eine wässrige Lösung eines ethylenisch ungesättigten Säuregruppentragenden, gegebenenfalls teilneutralisierten Monomeren und eines vernetzenden Monomeren radikalisch Polymerisiert wird,
   - das Polymerisat anschließend getrocknet und gemahlen wird,
   - das Polymerisat vor oder nach der Trocknung oberflächenvernetzt wird,

   **dadurch gekennzeichnet, dass** Cyclodextrine oder deren Derivate in Substanz oder in einem Lösungsmittel gelöst

   - der Monomerlösung vor deren Polymerisation zugesetzt werden, oder
   - auf das nicht getrocknete Polymergel aufgebracht werden, oder
   - auf das getrocknete und zerkleinerte Polymergel aufgetragen werden, wobei die Cyclodextrine oder deren Derivate gegebenenfalls während der Oberflächenvernetzung zugegeben werden, oder
   - auf das zerkleinerte und bereits oberflächenvernetzte Polymerisat aufgebracht werden.

11. Verfahren nach Anspruch 10, **dadurch gekennzeichnet, dass** die Menge an Cyclodextrinen oder Cyclodextrinderivaten 0,01 bis 10 Gew.-%, bezogen auf die Menge Polymerisat, beträgt.

12. Verfahren nach Anspruch 10 oder 11, **dadurch gekennzeichnet, dass** die Cyclodextrine oder Cyclodextrinderivate kovalent oder ionisch mit dem Polymer gebunden sind.

## Claims

1. Absorptive polymer based on optionally partly neutralized, monoethylenically unsaturated monomers which bear acid groups, the surface of which has been postcrosslinked after the polymerization,
   obtainable by a process in which

   - an aqueous solution of an ethylenically unsaturated, optionally partly neutralized monomer bearing acid groups and of a crosslinking monomer is free-radically polymerized,
   - the polymer is then dried and ground,
   - the polymer is surface crosslinked before or after the drying,

   **characterized in that** cyclodextrins or derivatives thereof, in substance or dissolved in a solvent,

- are added to the monomer solution before the polymerization thereof, or
- are applied to the undried polymer gel, or
- are applied to the dried and comminuted polymer gel, the cyclodextrins or derivatives thereof optionally being added during the surface crosslinking, or
- are applied to the comminuted and already surface crosslinked polymer.

2. Absorptive polymer according to Claim 1, **characterized in that** the extractable proportion of cyclodextrins is less than 85% of the amount present in the product.

3. Absorptive polymer according to Claim 1 or 2, **characterized in that** the extractable proportion of cyclodextrins is less than 60% of the amount present in the product.

4. Absorptive polymer according to any of the preceding claims, **characterized in that** the extractable proportion of cyclodextrins is less than 45% of the amount present in the product.

5. Absorptive polymer according to any of the preceding claims, **characterized in that** the amount of cyclodextrins or cyclodextrin derivatives is 0.01 to 10% by weight, based on the amount present in the polymer.

6. Absorptive polymer according to any of the preceding claims, **characterized in that** the cyclodextrins are $\alpha$-, $\beta$- or $\gamma$-cyclodextrins.

7. Absorptive polymer according to any of the preceding claims, **characterized in that** the cyclodextrins or cyclodextrin derivatives are bonded covalently or ionically to the polymer.

8. Construction for absorbing liquids, comprising an absorptive polymer according to any of the preceding claims.

9. Construction according to Claim 8, **characterized in that** the construction is a nappy or an incontinence product.

10. Process for preparing an absorptive polymer based on optionally partly neutralized, monoethylenically unsaturated monomers bearing acid groups, the surface of which has been postcrosslinked after the polymerization, in which

- an aqueous solution of an ethylenically unsaturated, optionally partly neutralized monomer bearing acid groups and of a crosslinking monomer is free-radically polymerized,
- the polymer is then dried and ground,
- the polymer is surface crosslinked before or after the drying,

**characterized in that** cyclodextrins or derivatives thereof, in substance or dissolved in a solvent,

- are added to the monomer solution before the polymerization thereof, or
- are applied to the undried polymer gel, or
- are applied to the dried and comminuted polymer gel, the cyclodextrins or derivatives thereof optionally being added during the surface crosslinking, or
- are applied to the comminuted and already surface crosslinked polymer.

11. Process according to Claim 10, **characterized in that** the amount of cyclodextrins or cyclodextrin derivatives is 0.01 to 10% by weight, based on the amount of polymer.

12. Process according to Claim 10 or 11, **characterized in that** the cyclodextrins or cyclodextrin derivatives are bonded covalently or ionically to the polymer.

## Revendications

1. Polymère absorbant à base de monomères à insaturation monoéthylénique, éventuellement partiellement neutralisés, portant des groupes acides, dont la surface a été post-réticulée après la polymérisation,
pouvant être obtenu par un procédé dans lequel

- on polymérise par voie radicalaire une solution aqueuse d'un monomère à insaturation éthylénique, éventuellement partiellement neutralisé, portant des groupes acides, et d'un monomère réticulable,
- le polymère est ensuite séché et broyé,
- le polymère est réticulé en surface avant ou après le séchage,

**caractérisé en ce que** des cyclodextrines ou leurs dérivés, tels quels ou dissous dans un solvant

- sont ajoutés à la solution de monomères avant la polymérisation, ou
- sont appliqués sur le gel de polymère non séché, ou
- sont appliqués sur le gel de polymère séché et fragmenté, les cyclodextrines ou leurs dérivés étant éventuellement ajoutés pendant la réticulation superficielle, ou
- sont appliqués sur le polymère fragmenté et déjà réticulé en surface.

**2.** Polymère absorbant selon la revendication 1, **caractérisé en ce que** la fraction extractible des cyclodextrines est inférieure à 85 % de la quantité présente dans le produit.

**3.** Polymère absorbant selon la revendication 1 ou 2, **caractérisé en ce que** la fraction extractible des cyclodextrines est inférieure à 60 % de la quantité présente dans le produit.

**4.** Polymère absorbant selon l'une quelconque des revendications précédentes, **caractérisé en ce que** la fraction extractible des cyclodextrines est inférieure à 45 % de la quantité présente dans le produit.

**5.** Polymère absorbant selon l'une quelconque des revendications précédentes, **caractérisé en ce que** la quantité des cyclodextrines ou dérivés de cyclodextrines vaut de 0,01 à 10 % en poids, par rapport à la quantité de polymère.

**6.** Polymère absorbant selon l'une quelconque des revendications précédentes, **caractérisé en ce que** les cyclodextrines consistent en des α-, β- ou γ-cyclodextrines.

**7.** Polymère absorbant selon l'une quelconque des revendications précédentes, **caractérisé en ce que** les cyclodextrines ou dérivés de cyclodextrines sont liés par liaison covalente ou ionique au polymère.

**8.** Construction destinée à l'absorption de liquides, comportant un polymère absorbant selon l'une quelconque des revendications précédentes.

**9.** Construction selon la revendication 8, **caractérisée en ce que** la construction est une couche ou un produit pour incontinence.

**10.** Procédé pour la préparation d'un polymère absorbant à base de monomères à insaturation éthylénique, éventuellement partiellement neutralisés, portant des groupes acides, dont la surface a été post-réticulée après la polymérisation, dans lequel

- on polymérise par voie radicalaire une solution aqueuse d'un monomère à insaturation monoéthylénique, éventuellement partiellement neutralisé, portant des groupes acides, et d'un monomère réticulable,
- le polymère est ensuite séché et broyé,
- le polymère est réticulé en surface avant ou après le séchage,

**caractérisé en ce que** des cyclodextrines ou leurs dérivés, tels quels ou dissous dans un solvant

- sont ajoutés à la solution de monomères avant la polymérisation, ou
- sont appliqués sur le gel de polymère non séché, ou
- sont appliqués sur le gel de polymère séché et fragmenté, les cyclodextrines ou leurs dérivés étant éventuellement ajoutés pendant la réticulation superficielle, ou
- sont appliqués sur le polymère fragmenté et déjà réticulé en surface.

**11.** Procédé selon la revendication 10, **caractérisé en ce que** la quantité des cyclodextrines ou dérivés de cyclodextrines vaut de 0,01 à 10 % en poids, par rapport à la quantité de polymère.

**12.** Procédé selon la revendication 10 ou 11, **caractérisé en ce que** les cyclodextrines ou dérivés de cyclodextrines sont liés par liaison covalente ou ionique au polymère.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0483380 A
- US 5360899 A
- US 5357012 A
- DE 19520989 A
- EP 806195 A
- WO 9422501 A
- WO 9422500 A
- US 5538655 A
- DE 19533269 A
- DE 4440236
- US 3740391 A
- US 4153585 A
- US 4638058 A
- US 5134127 A
- US 3453257 F
- US 4286082 A
- DE 2706135
- US 4076663 A
- DE 3503458
- DE 4020780
- DE 4244548
- DE 4323001
- DE 4333056
- DE 4418818
- US 4654039 A
- US 5314420 A
- US 5154713 A
- DE 3544770
- EP 317106 A
- WO 949043 A
- US 4666983 A


### In der Beschreibung aufgeführte Nicht-Patentliteratur


- **A. Zlatkis et al.** *Anal. Chem.,* vol. 45, 763ff
- **R. H. Waring.** *Xenobiotika,* vol. 17, 1363ff
- **A. Norberg et al.** *Gerontology,* 1984, vol. 30, 261 ff
- **J. Szejtli.** Cyclodextrin Technology. Kluwer Academic Publishers, 1988
- **G. Wenz.** *Angew. Chem.,* 1994, vol. 106, 851
- **Harada et al.** *J.Org.Chen,* 1993, vol. 58, 7524-28
- **A. P. Croft ; R. A. Bartsch.** *Tetrahedron,* vol. 39 (9), 1417-1473
- **Ch. Roussel ; A. Favrou.** *Journal of Chromatography A,* 1995, vol. 704, 67-74
- **T. Takeuchi ; T. Miwa.** *Chromatographia,* 1994, vol. 38, 453